(19) Europäisches Patentamt
European Patent Office
Office européen des brevets

(11) Veröffentlichungsnummer: **0 045 375**
**B1**

(12) # EUROPÄISCHE PATENTSCHRIFT

(45) Veröffentlichungstag der Patentschrift: **23.01.85**

(21) Anmeldenummer: **81105000.4**

(22) Anmeldetag: **27.06.81**

(51) Int. Cl.⁴: **C 07 D 215/22,** C 07 D 471/04 // (C07D471/04, 221/00, 221/00),(C07D471/04, 237/00, 221/00),(C07D471/04, 239/00, 221/00),(C07D471/04, 241/00, 221/00),(C07D471/04, 253/00, 221/00)

(54) **Verfahren zur Herstellung von Chinolinen, Naphthyridinen und anderen Stickstoff-bi-heterocyclen.**

(30) Priorität: **28.07.80 DE 3028520**

(43) Veröffentlichungstag der Anmeldung: **10.02.82 Patentblatt 82/6**

(45) Bekanntmachung des Hinweises auf die Patenterteilung: **23.01.85 Patentblatt 85/4**

(84) Benannte Vertragsstaaten: **AT BE CH DE FR GB IT LI LU NL SE**

(56) Entgegenhaltungen:
**AT - B - 337 700**
**DE - A - 2 341 146**

**THE JOURNAL OF THE AMERICAN CHEMICAL SOCIETY, Vol. 68, Juli 1946 CHARLES C. PRICE "The Synthesis of 4-Hydroxy-quinolines. I." Seiten 1204-1208**
**THE JOURNAL OF THE AMERICAN CHEMICAL SOCIETY; Vol. 68, Jänner 1946 A.R. SURREY "Some 7-Substituted 4-Aminoquinoline Derivatives" Seiten 113-116**
**JOURNAL OF MEDICINCAL CHEMISTRY, Vol. 21, No. 1, Jänner 1978 AI JENG LIN "Synthesis and Antitumor Activity of Halogen-Substituted 4-(3,3-Dimemthyl-1-triazeno)quinolines" Seiten 268-272**
**JOURNAL OF HETEROCYCLIC CHEMISTRY, Vol. 11, Dezember 1974 G.C. WRIGHT "Synthesis and Structural**

(73) Patentinhaber: **DYNAMIT NOBEL AKTIENGESELLSCHAFT, Postfach 1209, D-5210 Troisdorf, Bez. Köln (DE)**

(72) Erfinder: **Steffen, Klaus-Dieter, Dr., Röckelstrasse 36, D-5202 Hennef 1 (DE)**

(56) Entgegenhaltungen: (Fortsetzung)
**Assignement of 5,6-Dihydro-8-hydroxy-9-methoxy-1H, 7H-benzo(ij)quinolozine-1,7-dione" Seiten 849-851**
**LIEBIGS ANNALEN DER CHEMIE, Heft 4, April 1979, Weinheim P. MESSINGER "Darstellung von 5-Hydroxy-1,7-naphthyridin" Seiten 443-445**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein neues Verfahren zur Herstellung von Chinolinen, Naphthyridinen und anderen Stickstoff-bi-heterocyclen der allgemeinen Formel

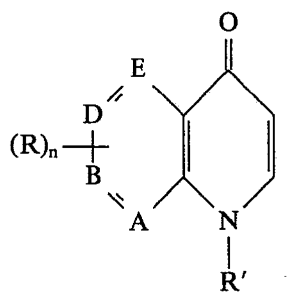

(I)

Diese Stickstoff-bi-heterocyclen sind aus der Literatur bekannt.

Einen Überblick über verschiedene Synthesen, besonders von 4,7-Dichlorchinolin, gibt Organ. Synthesis, Vol. III, S. 272:

So wird nach J. Amer. Chem. So., 68 (1946), 113, 4-Hydroxy-7-halogen-chinolin aus dem entsprechenden 2-Carbethoxy-Derivat und nach J. Amer. Chem. Soc., 68 (1946), 1204, 4-Hydroxy-7-chlor-chinolin aus dem 3-Carbaethoxy-Derivat in zwei getrennten Stufen durch alkalische Hydrolyse, Filtration der Carbonsäure und deren Dekarboxylierung in Mineralöl bzw. des Feststoffes hergestellt.

Nach J. Med. Chemistry, 21 (1978), 268, werden die 6-, 7- oder 8-Halogen-4-hydroxy-chinoline und nach J. Heterocycl. Chem., 11 (1974), 849, in 6- und 7-Stellung methoxy- bzw. äthoxylierte 4-Hydroxychinoline wiederum durch alkalische Verseifung und durch Dekarboxylierung in hochsiedenden Lösungsmitteln nach Zwischenisolierung der Säure hergestellt.

Auch 4-Oxo-6-methyl-6,7,8,9-tetrahydro-homopyrimidazol wurde durch alkalische Verseifung und anschließende Dekarboxylierung nach Arzneimittelforschung, 22 (1972), 815, synthetisiert.

Aus dem US-Patent 3 856 800 geht hervor, daß 4-Hydroxy-7-methyl-naphthyridin-1,8 über Vorstufen eines N-Oxids, dessen Sauerstoff am Ende wieder hydrogenolytisch entfernt werden muß, dargestellt werden kann.

Siedende 18%ige Salzsäure wurde zur Hydrolyse von 3-Carbäthoxy-4-hydroxy-6-methoxy-chinolin nach J. Amer. Chem. Soc., 68 (1946), 1204, sowie von 5-Hydroxy-6-carbaethoxy-naphthyridin-1,7, Liebigs Ann. Chemie, 4 (1979), 443, eingesetzt.

Bei der letzten Verbindung trat auch Dekarboxylierung zum 5-Hydroxy-naphthyridin-1,7 ein, das anschließend durch Sublimation gereinigt werden mußte.

Diesen bekannten Herstellungsverfahren ist gemeinsam, daß mit mindestens stöchiometrischen Mengen an Alkali oder Salzsäure gearbeitet wird, die neutralisiert werden müssen, was zu großen Mengen an Salzlösungen führt und heute zwangsläufig Entsorgungsprobleme mit sich bringt.

Weiterhin ist nachteilig, daß die Synthesen zweistufig sind durch die Isolierung und Waschung der Säure vor der Dekarboxylierung, was bei einer Übertragung in den Produktionsmaßstab hohe Kosten verursacht.

Es bestand also die Aufgabe, ein Verfahren zu finden, das wenig Abfälle entstehen läßt, einfach durchführbar ist und trotzdem gute Ausbeuten und Reinheiten der Endprodukte ergibt.

Die Lösung dieser Aufgabe wird nachfolgend beschrieben.

Der Ausgangsstoff (II) wird durch Ringschluß von Verbindung (III) unter Abspaltung von Äthanol oder Methanol

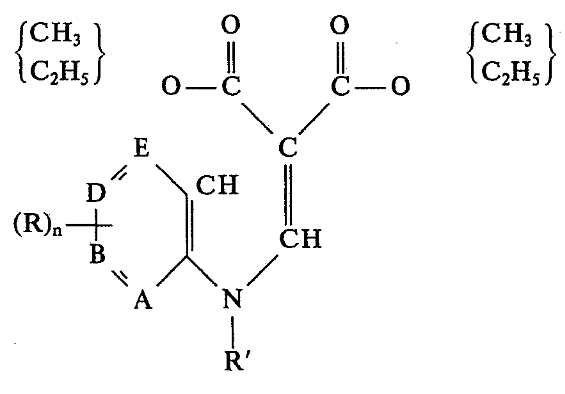

(III)

bei hohen Temperaturen von ca. 240—350° C in einigen Minuten hergestellt. Als Lösungsmittel werden hochsiedende Substanzen wie Diphenyläther, Dibenzylbenzol, Benzylbenzol, Diäthylphthalat, Polyphosphorsäure, Siliconöl, Triphenylmethan oder die verschiedensten handelsüblichen Paraffin- und Wärmeträgeröle verwendet, wie sie beispielsweise in den DE-OS 2 343 462 und 2 441 747 und der US-PS 3 149 104 und 3 673 193 beschrieben sind.

Gegenstand der Erfindung ist ein Verfahren zur Herstellung von Chinolinen, Naphthyridinen und anderen Stickstoff-bi-heterocyclen der allgemeinen Formel (I)

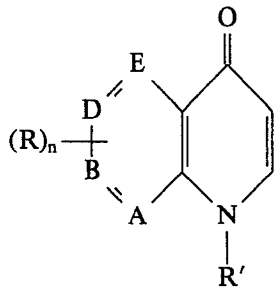

(I)

in der R = H, Halogen, Nitro-, Amino-, Keto-, Alkyl-, Alkylen-, Aryl- (ggf. substituierte Aryl-), Halogenalkylgruppen, über N, O, S oder $SO_2$ gebundene Aryl- oder Alkylsubstituenten in linearer oder cyclischer Anordnung sein können, R' = H, Aryl, Alkyl-, Alkylen-, Halogenalkyl-Substituenten darstellen, die Buchstaben A, B, D, E Kohlenstoff- oder bis 3 Stickstoffatome, die übrigen Kohlenstoffatome, sein können und n eine ganze Zahl von 0—4 einnehmen kann, welches dadurch gekennzeichnet, daß man in die bei ihrer Herstellung durch Cyclisierung erhaltenen Suspension der entsprechenden Carbaethoxy- oder Carbmethoxy-Verbindungen der allgemeinen Formel (II)

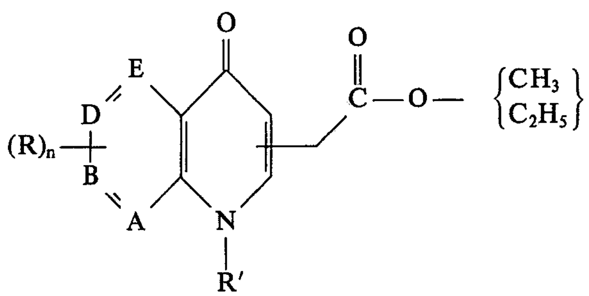

(II)

ohne vorherige Isolierung des Stoffes (II) durch Zugabe von Wasser mit Gehalten von sauren Katalysatoren in Konzentration von 0,05 bis 3,0 Gew.-% unter Druck verseift, dann die entstandene entsprechende Carbonsäure ohne ihre Isolierung zu (I) dekarboxyliert und ggf. jetzt isoliert oder direkt weitere Reaktionen anschließt.

Bei der Verseifung der Ester zu den Carbonsäuren ist Wasser in mindestens stöchiometrischer Menge zuzugeben. Der Ester ist in einem der genannten hochsiedenden Flüssigkeiten suspendiert oder gelöst.

Der aus der Cyclisierungsreaktion stammende, in einem Hochsieder suspendierte Ester (II) wird ohne Zwischenisolierung in einem Autoklaven mit Wasser im Vol.-Verhältnis von 1 : 0,5 bis 1 : 5 ($H_2O$ : Suspension), vorzugsweise 1 : 1,0 bis 1 : 3,0 versetzt. In dem Wasser sind 0,05 bis 3,0, vorzugsweise 0,3 bis 0,6 Gew.-% eines sauren Katalysators wie Schwefelsäure, p-Toluolsulfonsäure, Phosphorsäure, Salzsäure gelöst. Auch saure Ionenaustauscher sind bei genügender thermischer Stabilität einsetzbar. Unter allmählicher Abdestillation von Alkohol wid bei 120 bis 250, vorzugsweise 150 bis 180°C, bei 4 bis 8 bar in 4 bis 8 h verseift. Die Reaktion ist beendet, wenn nur noch Wasser abdestilliert. Daraufhin wird auf etwa 80 bis 95°C abgekühlt, die zur Neutralisation des verwendeten sauren Katalysators notwendige Menge an wäßriger, konzentrierter Alkalilauge, besonders Natronlauge oder Kalilauge zugesetzt und alles Wasser bei allmählich steigenden Temperaturen abdestilliert. Auch hier erfolgt keine Zwischenisolierung der entstandenen Carbonsäure. Nach Erreichen einer Sumpftemperatur von etwa 190 bis 210°C beginnt die Dekarboxylierung, d. h. es wird $CO_2$ frei. Die Temperatur wird weiter auf 230 bis 240°C gesteigert, bei dieser Temperatur in der Regel 3 bis 8 Stunden belassen, bis durch Beendigung der $CO_2$-Entwicklung die Reaktion abgeschlossen ist.

Die Suspensionskonzentration von (II) in dem Hochsieder, die zwischen 2 Gew.-% und 15 Gew.-% variieren kann, die Katalysatorkonzentration und die Reaktionszeit sowie -temperatur sind dabei einander beeinflussende Größen. Eine höhere Konzentration von (II) erfordert höhere Katalysatorkonzentrationen oder schärfere Reaktionsbedingung, d. h. höhere Temperatur und gegebenenfalls längere Reaktionszeit.

Zur besseren Benetzung des Hochsieders mit Wasser empfiehlt es sich, ein oder mehrere Tropfen eines handelsüblichen Benetzungs- oder Entspannungsmittels zuzugeben. Von Vorteil kann auch sein, Phasentransfer-Katalysatoren wie Benzyltriäthyl-, Benzyltrimethyl-ammoniumchlorid, Tetramethylammoniumchlorid, Benzyltriphenylphosphoniumchlorid dem Wasser zuzusetzen.

Es ist weiterhin anzuraten, die Katalysatorsäure nach beendeter Verseifung zu neutralisieren, da sonst das dekarboxylierte Endprodukt dunkel gefärbt wird.

Wenn nach dieser mehrstufigen Eintopfreaktion das Endprodukt im Wärmeträger vorliegt, kann direkt eine weitere Reaktion angeschlossen werden, wie z. B. die Chlorierung von HCC mit Phosphoroxychlorid, wie sie in Org. Synth.,Vol. III, S. 272, beschrieben ist, und erst dann ist in der dort beschriebenen Art und Weise aufzuarbeiten. Gegebenenfalls ist nach erfolgter Dekarboxylierung die Verbindung (I) zu isolieren.

Da die Hochsieder durch Trocknen nur sehr schlecht zu entfernen sind, muß mit leichter flüchtigen Lösungsmitteln nachgewaschen werden. Als solche Lösungsmittel bieten sich an die aliphatischen und aromatischen Kohlenwasserstoffe, wie die Benzine mit $C_5$—$C_{10}$, Toluol, Xylole, Petroläther; Alkohole, wie Methanol, Äthanol, Methoxyäthanol, Propanol, Butanol; Ketone, wie Aceton, Butanon. Zur restlosen Entfernung der Katalysatorsalze empfiehlt es sich — besonders bei wenig lösenden, unpolaren Lösungsmitteln — eine Waschung mit Wasser anzuschließen.

Diese Lösemittel können, soweit sich darin (I) schlecht löst, auch als Fällungsmittel für (I) aus dem Hochsieder eingesetzt werden und so im Volumen-Verhältnis von Suspension : Flüssigmittel = 1 : 0,1 bis 1 : 10 angewandt werden. Dadurch erhöht sich die Ausbeute an (I) meist aber zu Lasten der Reinheit.

Besonders vorteilhaft hat sich erfindungsgemäß die Auskristallisation von (I) durch Abkühlen, Filtration und Nachwaschen mit Aceton, bis alle Hochsieder entfernt sind und anschließendes Waschen mit wenig Wasser erwiesen. Dadurch werden gute Ausbeuten von 75—90% d. Th. sowie sehr gute Reinheiten bis 99% und darüber erzielt.

Diese Art Aufarbeitung kann dann von großer Wichtigkeit sein, wenn stellungsisomere Verbindungen des gleichen Molekulargewichts in kleinen Konzentrationen bei Folgereaktionen oder bei der Anwendung stören, z. B. 4-Hydroxy-5-chlorchinolin im 4-Hydroxy-7-chlor-chinolin.

Die verwendeten Lösungsmittel wie die Hochsieder, die bei den Reaktionen eingesetzt werden, oder die Tiefsieder, die zum Waschen von (I) verwendet werden, können nach ein- oder mehrmaligem Gebrauch aufdestilliert und stets wieder benutzt werden. Die in der Menge sehr gering anfallenden Destillationssümpfe können verbrannt werden.

Demnach wurde die gestellte Aufgabe löst. Nur geringe Mengen an Nebenprodukten und Salzen entstehen und können einfach entfernt werden. Überraschend ist nur eine katalytisch wirksame Menge Säure zur Verseifung erforderlich und die Reaktion einstufig ohne Isolierung der Zwischenprodukte ausführbar.

Die erstaunlich hohen Ausbeuten und Reinheiten dieses mehrstufigen Eintopfverfahrens übertreffen weit die Gesamtausbeute von (I), bezogen auf (II) der bekannten mehrstufigen Verfahren.

Die Verfahrensprodukte sind wichtige Zwischenprodukte für die Arzneimittelsynthese. So bekannte Produkte wie Chloroquin, Glafenin oder Nalidixinsäure können daraus hergestellt werden.

Verwendete Abkürzungen:

CHCC: 3-Carbaethoxy-4-hydroxy-7-(oder 5-)chlor-chinolin
HCC: 4-Hydroxy-7-(oder 5-)chlor-chinolin
PMME: α-Picolyl-aminomethylen-malonsäurediäthylester
CHMN: 3-Carbaethoxy-4-hydroxy-7-methyl-naphthyridin-1,8
HMN: 4-Hydroxy-7-methyl-naphthyridin-1,8
p-TSS: p-Toluolsulfonsäure

## Beispiele 1 bis 4

Eine 5gew.-%ige Suspension von CHCC in Benzylbenzol wurde in einem mit Rührer, Temperaturanzeige, Manometer, Druckhalteventil mit nachgeschaltetem Kühler ausgerüsteten Stahlautoklaven gegeben und mit dem gleichen Volumen an Wasser, in dem in nachfolgender Tabelle aufgeführte saure Katalysatoren in verschiedenen Konzentrationen und einige Tropfen Entspannungsmittel gelöst waren, versetzt. Nach Schließen des Autoklaven wurde 8 Stunden auf 150°C aufgeheizt und allmählich Äthanol und Wasser abdestilliert. Danach wurde über Nacht abgekühlt, mit der für die Katalysatorsäure äquivalenten Menge 10gew.-%iger NaOH neutralisiert, und wieder drucklos aufgeheizt unter Abdestillation des restlichen Wassers. Die Innentemperatur wurde in 4 Stunden auf 230—240°C belassen, bis die $CO_2$-Entwicklung beendet war. Es wurde abgekühlt, mit dem doppelten Volumen an Heptan verdünnt, das HCC abfiltriert, mit Heptan nachgewaschen und getrocknet. Die Ausbeute bezieht sich auf das eingesetzte m-Chloranilin; die Reinheit ist in Flächen-% angegeben, ermittelt durch HPLC, d. h. Hochdruckflüssigkeitschromatographie.

| Beispiele | Katalysator Art | Menge [% gel. i. $H_2O$] | HCC-Ausb. [% d. Th.] | Reinheit [HPLC-Fl.-%] nicht umgesetzt | HCC-7 | HCC-5 |
|---|---|---|---|---|---|---|
| 1 | $H_3PO_4$ | 0,25 | 90,7 | 1,5 | 96,7 | 1,8 |
| 2 | $H_3PO_4$ | 0,50 | 90,1 | 0 | 97,8 | 2,3 |
| 3 | $H_2SO_4$ | 0,25 | 85,8 | 0,3 | 95,1 | 1,8 |
| 4 | p-Toluol-sulfonsäure | 0,125 | 85,6 | 0 | 98,0 | 1,0 |

Beispiele 5 bis 7

Wie in den Beispielen 1 bis 4 wurde eine 5gew.-%ige Suspension von CHCC in Benzylbenzol in einem Druckautoklav mit nur $^1/_3$ des Suspensionsvolumens an Wasser verdünnt, das in nachfolgender Tabelle aufgeführte wechselnde Mengen an p-Toluolsulfonsäure und einige Tropfen Netzmittel gelöst enthielt.

Es wurde 8 Stunden auf 150°C aufgeheizt unter Abdestillation von Äthanol und $H_2O$, dann die p-Toluolsulfonsäure mit NaOH neutralisiert, und drucklos auf 230 bis 240°C erhitzt, bis nach 4 Stunden die $CO_2$-Entwicklung beendet war. Nach dem Abkühlen wird die Reaktionsmischung mit dem doppelten Volumen Heptan verdünnt, das HCC abfiltriert, mit Heptan gewaschen und getrocknet.

| Beispiele | p-TSS-Konz. [% i. $H_2O$] | HCC-Ausbeute [% d. Th.] | Reinheit [HPLC-Fl.-%] nicht umgesetzt | HCC-7 | HCC-5 |
|---|---|---|---|---|---|
| 5 | 0,5 | 83,4 | 0,6 | 97,6 | 1,2 |
| 6 | 0,25 | 85,6 | 3,3 | 94,2 | 2,3 |
| 7 | 0,125 | 87,1 | 19,0 | 78,7 | 2,1 |

Beispiele 8 und 9

Wie in den Beispielen 1 bis 4 wurde eine 5gew.-%ige Suspension von CHCC in Benzylbenzol hergestellt und in einem VA-Autoklav mit nur der halben Menge an Wasser vesetzt, das 0,5 Gew.-% bzw. 0,25 Gew.-% an p-Toluolsulfonsäure und 1 Tropfen Netzmittel gelöst enthielt. Zur Verseifung wurde unter Druck und Abdestillation von Äthanol/$H_2O$ auf 160°C über 8 bzw. 4 Stunden aufgeheizt. Nach der Neutralisation der p-Toluolsulfonsäure mit 20gew.-%iger wäßriger KOH wurde 5 Stunden auf 230 bis 240°C aufgeheizt, gekühlt, mit Heptan verdünnt, HCC abfiltriert, nachgewaschen und getrocknet.

| Beispiele | p-TSS-Konz. [% i. $H_2O$] | Vers.-Zeit [h] bei 160°C | HCC-Ausb. [% d. Th.] | Reinheit [HPLC-Fl.-%] nicht umgesetzt | HCC-7 | HCC-5 |
|---|---|---|---|---|---|---|
| 8 | 0,5 | 8 | 89,3 | 0 | 98,4 | 1,6 |
| 9 | 0,25 | 4 | 91,8 | 1,1 | 97,0 | 1,8 |

Beispiele 10 bis 12

Gewaschenes und getrocknetes CHCC wurde nachträglich wieder in Dibenzylbenzol in Mengen von 4, 6 und 8 Gew.-% suspendiert und in einem Autoklaven mit dem halben Volumen an Wasser versetzt, das 0,5 Gew.-% p-Toluolsulfonsäure und 1 Tropfen Netzmittel gelöst enthielt. Nach der Verseifung bei

160° C in 5 Stunden wurde abgekühlt, die p-Toluolsulfonsäure neutralisiert, und die Dekarboxylierung bei 230° C in 5 Stunden durchgeführt. Die Ergebnisse sind in nachfolgender Tabelle festgehalten.

| Beispiele | CHCC-Konz. [%] | HCC-Ausbeute (% d. Th.] | Reinheiten [HPLC-Fl.-%] nicht umgesetzt | HCC-7 | HCC-5 |
|---|---|---|---|---|---|
| 10 | 4 | 81,4 | 1,1 | 97,5 | 0,9 |
| 11 | 6 | 88,0 | 13,6 | 85,3 | 0,9 |
| 12 | 8 | 86,6 | 16,6 | 82,5 | 0,8 |

Beispiele 13 bis 15

Bei diesen Beispielen wurde die Cyclisierungsreaktion nach DE-OS 2 343 462 so ausgeführt, daß am Ende eine 9,6- oder 4gew.-%ige Suspension von CHCC in Benzylbenzol vorlag. Jede der beiden Suspensionen wurde im Autoklaven mit dem halben Volumen an Wasser versetzt, das 0,3—0,4 Gew.-% p-Toluolsulfonsäure und 1 Tropfen Netzmittel gelöst enthielt. Für die Verseifung wurde 6 Stunden auf 170° C unter Abdestillation von Äthanol/$H_2O$ erhitzt, dann die p-Toluolsulfonsäure neutralisiert und die Dekarboxylierung in 6 Stunden bei 240° C durchgeführt. Das HCC wurde über Nacht auskristallisiert, filtriert, dann 3mal mit wenig Aceton und 2mal mit Wasser gewaschen und getrocknet. Ausbeute und Reinheiten sind in nachfolgender Tabelle aufgeführt.

| Beispiele | CHCC-Konz. [%] | HCC-Ausbeute [% d. Th.] | Reinheiten [HPLC-Fl.-%] nicht umgesetzt | HCC-7 | HCC-5 |
|---|---|---|---|---|---|
| 13 | 9 | 75,1 | Spuren | 99,5 | 0,2 |
| 14 | 6 | 76,2 | Spuren | 99,7 | 0,1 |
| 15 | 4 | 77,7 | Spuren | 99,2 | 0,3 |

Beispiel 16

Zunächst wurde eine Cyclisierungsreaktion zum CHCC durchgeführt, so daß am Ende eine 9gew.-%ige Suspension in Benzylbenzol vorlag. Nach Umfüllen in einen Autoklaven wurde die Suspension mit dem halben Volumen an Wasser vermischt, das 0,4gew.-%ige p-Toluolsulfonsäure und einige Tropfen Netzmittel gelöst enthielt. Es wurde in 6 Stunden bei 160° C unter Abdestillation von Äthanol/$H_2O$ verseift, der Katalysator neutralisiert, dann in 6 Stunden bei 230° C dekarboxyliert. Nach dem Erkalten wurde die Reaktionsmischung mit dem 2,5fachen Volumen an Heptan versetzt, das ausgefallene HCC abfiltriert, mit wenig Heptan und Aceton gewaschen und getrocknet.
Ausbeute: 85,0% d. Th.
Reinheit: 95,5% HCC-7, 0,8% HCC-5, 3,5% Nebenprodukte.

Beispiel 17

60 g PMME wurden in 1,05 l Dibenzylbenzol cyclisiert, so daß eine 4,0gew.-%ige Suspension an CHMN entstand. Diese wurde in einen Autoklaven überführt, mit 550 ml Wasser, in dem 0,5 Gew.-% p-Toluolsulfonsäure gelöst waren, vermischt und nach Zugabe von 1 Tropfen Netzmittel in 8 Stunden bei 170° C unter Abdestillation von Äthanol/$H_2O$ druckverseift. Nach Neutralisation der Katalysatorsäure wurde drucklos bei 240° C in ca. 5 Stunden zu HMN dekarboxyliert. Es wurde über Nacht abgekühlt, filtriert, erst mit Heptan, dann mit Aceton/$H_2O$ gewaschen und getrocknet.
Ausbeute: 29,7 g (86% d. Th.).
Fp.: 232—34° C (eine nochmals sublimierte Probe hatte einen Fp. von 236° C).
Reinheit (HPLC-Fl.-%): 97,5% HMN, 1,3% CHMN, 1,2% andere Nebenprodukte.

Beispiel 18

Eine 4%ige Suspension von 2-Chloro-5-oxo-6-carbäthoxy-5,8-dihydropyrido[2,3d] pyrimidin in Di benzylbenzol wird in der angegebenen Art und Weise unter Druck verseift und nach Abdestillation des überschüssigen Wassers zu 2-Chloro-5-oxo-5,8-dihydro-pyrido[2,3d]-pyrimidin

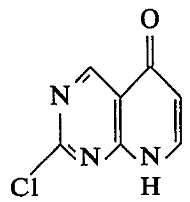

dekarboxyliert.

Dieses Chlor-pyridopyrimidin kann als solches isoliert werden oder die Chlorgruppe direkt mit Aminen wie Dialkylaminen, Pyrrolidin, N-monosubstituierten Piperazinen nach Zugabe einer Base zum Abfangen von HCl oder mit Alkalisalzen organischer Säuren oder mit Alkali-alkoholaten sowie -thioalkoholaten umgesetzt und dann erst isoliert werden.

**Patentansprüche**

1. Verfahren zur Herstellung von Chinolinen, Naphthyridinen und anderen Stickstoff-bi-heterocyclen der allgemeinen Formel (I)

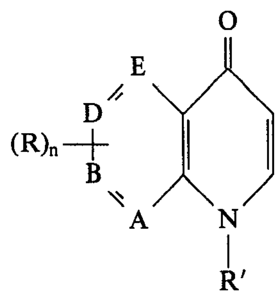

(I)

in der R = H, Halogen, Nitro-, Amino-, Keto-, Alkyl-, Alkylen-, Aryl- (ggf. substituierte Aryl-), Halogenalkylgruppen, über N, O, S oder $SO_2$ gebundene Aryl- oder Alkylsubstituenten in linearer oder cyclischer Anordnung sein können, R' = H, Aryl, Alkyl-, Alkylen-, Halogenalkyl-Substituenten darstellen, von den Buchstaben A, B, D, E bis 3 Atome Stickstoffatome, die übrigen Kohlenstoffatome sein können und n eine ganze Zahl von 0—4 einnehmen kann,
dadurch gekennzeichnet, daß man in die bei ihrer Herstellung durch Cyclisierung erhaltenen Suspension der entsprechenden Carbaethoxy- oder Carbmethoxy-Verbindungen der allgemeinen Formel (II)

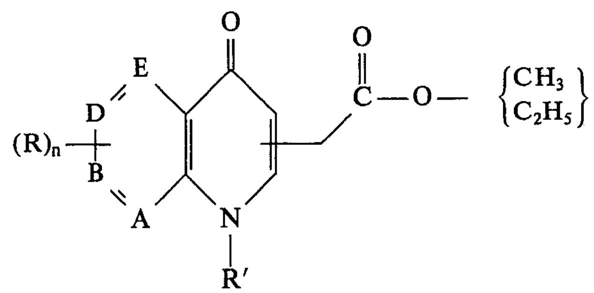

(II)

ohne vorherige Isolierung des Stoffes (II) durch Zugabe von Wasser mit Gehalten von sauren Katalysatoren in Konzentrationen von 0,05 bis 3,0 Gew.-% unter Druck verseift, dann die entstandene entsprechende Carbonsäure ohne ihre Isolierung zu (I) dekarboxyliert und gegebenenfalls jetzt isoliert oder direkt weitere Reaktionen anschließt.

2. Verfahren nach Anspruch 1, dadurch gekennzeichnet, daß man die Verseifung der Verbindungen (II) mit Wasser im Volumenverhältnis von $H_2O$ : Suspension der Verbindung (II) in einem Hochsieder = 1 : 0,5 bis 1 : 5 bei Temperaturen von 120 bis 250° C, unter Druck und gleichzeitiger Abdestillation von Alkohol vornimmt.

3. Verfahren nach den Ansprüchen 1 oder 2, dadurch gekennzeichnet, daß das Wasser saure

Katalysatoren wie Schwefelsäure, p-Toluolsulfonsäure, Phosphorsäure, Salzsäure in Konzentrationen von 0,3 bis 0,6 Gew.-% enthält.

4. Verfahren nach mindestens einem der Ansprüche 1 bis 3, dadurch gekennzeichnet, daß daß man die Katalysatorsäure nach beendeter Verseifung mit Alkali neutralisiert und vorhandenes Wasser abdestilliert.

5. Verfahren nach mindestens einem der Ansprüche 1 bis 4, dadurch gekennzeichnet, daß die Dekarboxylierung bei Temperaturen von 190 bis 240° C erfolgt.

6. Verfahren nach mindestens einem der Ansprüche 1 bis 5, dadurch gekennzeichnet, daß man das Endprodukt mit Lösungsmitteln wie Kohlenwasserstoffen, Alkoholen oder Ketonen und ggf. Wasser, vorzugsweise mit Aceton und Wasser, nachwäscht.

**Claims**

1. Process for the production of quinolines, naphthyridines and other nitrogen-biheterocyclics of the general formula (I)

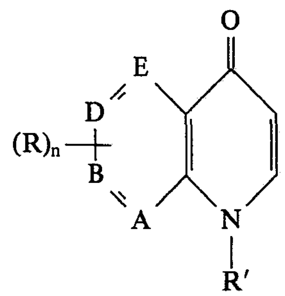

(I)

in which R=H, halogen, nitro-, amino-, keto-, alkyl-, alkylene-, aryl- (optionally substituted aryl-) groups or can be aryl- or alkyl- substituents bound in linear or cyclic arrangement through N, O, S or $SO_2$, R' represents H, aryl, alkyl-, alkylene- or halogenoalkylene-substituents, the letters A, B, D, E can be carbon or up to 3 nitrogen atoms, the remainder carbon atoms, and n can include a whole number of 0 to 4,

characterised in that the carbethoxy- or carbomethoxy- compounds of the general formula (II) corresponding thereto

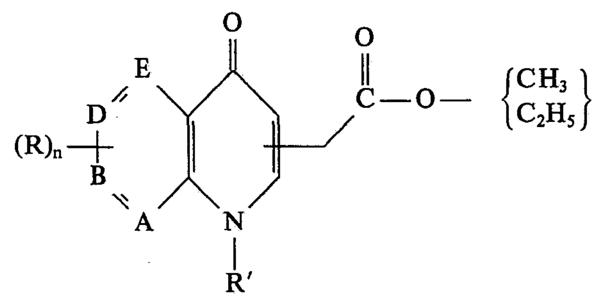

(II)

are saponified under pressure by addition of water with contents of acid catalysts in concentrations of 0.05 to 3.0% by weight to the suspension obtained on production thereof by cyclisation without previous isolation of substance (II), then the corresponding carboxylic acid which exists is decarboxylated to (I) without its isolation and optionally is then isolated or subjected directly to further reactions.

2. Process according to claim 1, characterised in that the saponification of the compounds (II) with water is undertaken in a volume ratio of $H_2O$ : suspension of compound (II) in a high boiling material = 1 : 0.5 to 1 : 5, at temperatures of 120 to 250° C, under pressure and simultaneous distillation off of alcohol.

3. Process according to claims 1 or 2, characterised in that the water contains acid catalysts, such as sulphuric acid, p-toluenesulphonic acid, phosphoric acid or hydrochloric acid in concentrations of 0.3 to 0.6% by weight.

4. Process according to at least one of claims 1 to 3, characterised in that the catalyst acid is neutralized with alkali at the end of the saponification and water present is distilled off.

5. Process according to any one of claims 1 to 4, characterised in that the decarboxylation is achieved at temperatures of 190 to 240° C.

6. Process according to at least one of claims 1 to 5, characterised in that the end product is after-washed with solvents such as hydrocarbons, alcohols or ketones and if necessary water, preferably with acetone and water.

## Revendications

1. Procédé de préparation de quinoléines, de naphtyridines et autres bi-hétérocycles azotés de formule générale (I):

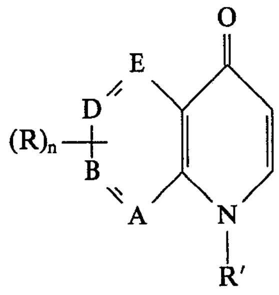

(I)

[dans laquelle R représente un atome d'hydrogène ou d'halogène ou un groupe nitro, amino, céto, alkyle, alkylène, aryle (éventuellement aryle substitué) halogénoalkyle, les substituants aryles ou alkyles, reliés par l'intermédiaire de N, O, S ou $SO_2$, pouvant être en configuration linéaire ou cyclique, R' représente H, un substituant aryle, alkyle, alkylène ou halogénoalkyle, le lettres A, B, D, E pouvant représenter jusqu'à 3 atomes d'azote et les autres étant des atomes de carbone, et n étant un nombre entier pouvant valoir de 0 à 4],
procédé caractérisé en ce que, dans la suspension, obtenue par cyclisation lors de leur préparation, des composés carbéthoxylés ou carbométhoxylés correspondants de formule générale (II):

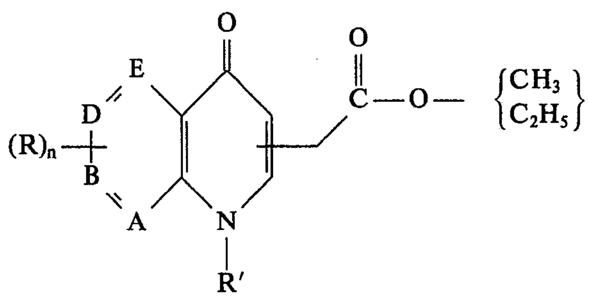

(II)

on saponifie sous pression, sans isolement préalable de la substance (II), par addition d'eau contenant des catalyseurs acides présents en une concentration de 0,05 à 3,0% en poids, puis, sans l'isoler, on soumet l'acide carboxylique correspondant résultants à une décarboxylation pour obtenir (I) et, le cas échéant, on l'isole ou effectue directement d'autres réactions.

2. Procédé selon la revendication 1, caractérisé en ce qu'on effectue sous pression, en chassant simultanément par distillation l'alcool, la saponification des composés (II) en utilisant de l'eau selon un rapport volumétrique de $H_2O$ : suspension du composé (II) dans une substance à point élevé d'ébullition de 1 : 0,5 à 1 : 5, en opérant à des températures de 120 à 250° C.

3. Procédé selon les revendications 1 ou 2, caractérisé en ce que l'eau contient, en des concentrations de 0,3 à 0,6% en poids, des catalyseurs acides comme l'acide sulfurique, l'acide p-toluènesulfonique,, l'acide phosphorique, l'acide chlorhydrique.

4. Procédé selon l'une au moins des revendications 1 à 3, caractérisé en ce qu'après achèvement de la saponification, on neutralise à l'aide d'une substance alcaline l'acide ayant servi de catalyseur et l'on chasse par distillation l'eau présente.

5. Procédé selon l'une au moins des revendications 1 à 4, caractérisé en ce que la décarboxylation est réalisée à des températures de 190 à 240° C.

6. Procédé selon l'une au moins des revendications 1 à 5, caractérisé en ce qu'on soumet le produit final à un lavage subséquent avec des solvants comme des hydrocarbures, des alcools ou des cétones et éventuellement l'eau, de préférence en utilisant de l'acétone et de l'eau.